(19) Europäisches Patentamt
European Patent Office
Office européen des brevets

(11) **EP 2 618 144 A1**

(12) **EUROPÄISCHE PATENTANMELDUNG**

(43) Veröffentlichungstag:
24.07.2013 Patentblatt 2013/30

(51) Int Cl.:
*G01N 33/487* (2006.01)

(21) Anmeldenummer: **12000258.9**

(22) Anmeldetag: **18.01.2012**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Medical Device UG**
**429 Rheine (DE)**

(72) Erfinder:
• **Bartetzko, Norbert**
**48301 Nottuln (DE)**
• **Bartetzko, Robert**
**59071 Hamm (DE)**

(54) **Verfahren und Behälter zum Beladen eines Messgeräts mit Messmitteleinheiten**

(57) Die vorliegende Erfindung betrifft ein Verfahren und einen Behälter (5) zum Beladen eines Messgeräts (3) mit einer Messmitteleinheit (21), sowie ein Kit (1) bestehend aus einem Messgerät (3) und einem Behälter (5). Das Messgerät (3) wird dabei zumindest teilweise in den Behälter (5) eingeführt und entnimmt innerhalb des Behälters (5) eine der in dem Behälter gelagerten Messmitteleinheiten (21) und wird mit der entnommenen Messmitteleinheit (21) wieder aus dem Behälter (5) herausgeführt. Dadurch wird ein außerordentlich sicheres, hygienisches und wirtschaftliches Beladen eines Messgeräts (3) mit einer Messmitteleinheit (21) erreicht.

**Fig. 1**

EP 2 618 144 A1

**Beschreibung**

Die vorliegende Erfindung betrifft ein Verfahren und einen Behälter zum Beladen eines Messgeräts mit Messmitteleinheiten. Insbesondere betrifft die Erfindung einen Behälter für eine Mehrzahl von Messmitteleinheiten in Form von Teststreifen zum Beladen eines Blutzuckerspiegelmessgeräts mit einem der Teststreifen für eine Messung des Blutzuckerspiegels eines Benutzers.

Das Überwachen des Blutzuckerspiegels ist ein wichtiger und regelmäßiger Bestandteil des Lebens vieler Diabetiker. Richtige Messergebnisse und ein mehrmaliges Testen an jedem Tag sind wichtige Voraussetzungen für ein beschwerdefreies Leben mit der Diabetes-Krankheit. Ein fehlerhaftes Verhalten des Diabetikers kann ernsthafte Komplikationen wie z.B. kardiovaskuläre Erkrankungen, Nervenschädigungen und Blindheit hervorrufen.

Die meisten tragbaren Messgeräte, mit denen ein Benutzer selbständig den eigenen Blutzuckerspiegel messen kann, beruhen auf einer amperometrischen Bestimmung der Glucose-Konzentration im Blut. Ein Teststreifen weist dabei einen saugfähigen Abschnitt zum Aufnehmen eines Tropfens Körperflüssigkeit, wie etwa Blut oder Tränenflüssigkeit, auf, der über eine Kapillare die Körperflüssigkeit in einem Sensorbereich leitet. In dem Sensorbereich reagiert die Körperflüssigkeit mit dem Enzym Glucose-Oxidase und schließt einen elektrischen Kontakt zwischen zwei im Teststreifen befindlichen Elektroden. Jede Elektrode ist jeweils mit einer an einem Ende des Teststreifens befindlichen Kontaktfläche leitend verbunden. Das mit den Kontaktflächen versehene Ende des Teststreifens wird vor der Messung in ein passendes Messgerät gesteckt, wo Kontaktköpfe auf die Kontaktflächen greifen. Nachdem auf dem aus dem Messgerät herausragenden saugfähigen Abschnitt des Teststreifens ein Tropfen Körperflüssigkeit aufgetragen wurde, kann über eine vom Messgerät an den Elektroden angelegte Spannung ein Stromverlauf gemessen werden, der für die Glucose-Konzentration charakteristisch ist. Ein Teststreifen ist nach einer Messung verbraucht, sodass für jede Messung ein neuer, trockener und sauberer Teststreifen verwendet werden muss.

Schaut man sich die verschiedenen Blutzuckerspiegelmessgeräte genauer an, dann stellt man fest, dass sie sich zwar in vielen Details unterscheiden, im Prinzip aber in zwei Gruppen eingeteilt werden können: Dosen-Systeme und integrierte Systeme.

Bei Dosen-Systemen werden die Teststreifen in einer vom Messgerät getrennten Dose aufbewahrt. Der Anwender entnimmt manuell einen Teststreifen und legt diesen ebenfalls manuell in das Messgerät ein.

Die Dokumente EP 1 352 611 B1, EP 1 854 738 B1, US 6,508,380 B1 und WO 2008/125244 A1 beschreiben Dispenser-Dosen, die für das Lagern und Ausgeben von Teststreifen konstruiert wurden. Dabei wird der oberste Teststreifen so weit aus der Öffnung des Dispensers herausgeschoben, dass er sich mit den Fingern ergreifen lässt und ganz herausgezogen werden kann. Dann wird der Teststreifen manuell in das Messgerät eingeführt.

Vorteil dieser Systeme ist, dass das Messgerät sehr kompakt bleiben kann. Nachteilig ist allerdings, dass das manuelle Beladen des Messgeräts unhygienisch ist, da der einzulegende Teststreifen durch die Berührung verschmutzt werden kann und ggf. Feuchtigkeit aufnimmt, was wiederum das Messergebnis beeinträchtigt. Ein am Teststreifen vorgesehener Berührabschnitt hat wiederum unerwünscht größere Teststreifen zur Folge. Im Übrigen haben viele Diabetiker ein hohes Alter und sind in ihrer Fingermotorik stark eingeschränkt, sodass ein manuelles Beladen oft ganz ausscheidet. Die Teststreifen müssen insgesamt recht groß ausgestaltet werden, damit selbst bei guter Fingermotorik ein benutzerfreundliches Beladen möglich ist. Am Markt hat sich eine Mindestlänge der Teststreifen von 27 mm herausgestellt. Für eine zuverlässige Messung gemäß DIN EN ISO 15197 ist diese Mindestlänge allerdings gar nicht notwendig.

Bei sogenannten "integrierten Systemen" ist der Teststreifenbehälter in dem Messgerät in Form einer Trommel oder Diskette integriert bzw. einlegbar. Diese Systeme sind hygienischer und können mit kleineren Teststreifen beladen werden. Es entfällt die Vorbereitung jeder einzelnen Messung und man muss nicht die verschiedenen Komponenten einzeln mit sich führen. Man muss bei Bedarf lediglich die entleerte Trommel oder Diskette gegen eine gefüllte austauschen. Nachteilig an den integrierten Systemen ist allerdings, dass sie eine relativ aufwändige Mechanik zum Beladen des Messgeräts mit einem Teststreifen benötigen und daher beispielsweise zum Mitnehmen für unterwegs zu groß und schwer sind. Außerdem sind die aufwändigen Mechaniken störanfällig.

Die EP 2 157 428 A1 beschreibt einen Dispenser, der in einem "stand-alone"-Modus und einem "attached-to-meter"-Modus funktionieren kann. Im "standalone"-Modus gibt der Dispenser einen Teststreifen zur herkömmlichen manuellen Entnahme aus. Im "attached-to-meter"-Modus gibt der Dispenser nach einem Andokken an ein Messgerät die Teststreifen unter Betätigung eines Schiebers aus und führt diesen mit dem Schieber in das Messgerät ein. Nachteilig daran ist, dass das Betätigen des Schiebers bei angedocktem Dispenser umständlich und bei eingeschränkter Fingermotorik ggf. ganz unmöglich ist.

Es ist daher die Aufgabe der vorliegenden Erfindung, ein Verfahren und einen Behälter zum Beladen eines Messgeräts mit einer Messmitteleinheit bereitzustellen, wodurch ein hygienischeres, wirtschaftlicheres und benutzerfreundlicheres Beladen ermöglicht wird.

Gelöst wird diese Aufgabe durch ein Verfahren und einen Behälter gemäß den Merkmalen des Anspruchs 1 bzw. 6, sowie ein Kit gemäß den Merkmalen des Anspruchs 13. Vorteilhafte Ausführungsformen der Erfindung sind den jeweiligen Unteransprüchen bzw. der Beschreibung und den Figuren zu entnehmen.

Gemäß einem ersten Aspekt der Erfindung wird ein Verfahren zum Beladen eines Messgeräts mit einer Messmitteleinheit bereitgestellt mit den folgenden Schritten:

- Einführen zumindest eines Teils des Messgeräts in einen eine Mehrzahl von Messmitteleinheiten enthaltenden Behälter,
- Entnehmen einer der Messmitteleinheiten mittels des Messgeräts innerhalb des Behälters,
- Herausführen des zumindest einen Teils des Messgeräts aus dem Behälter mit der entnommenen Messmitteleinheit.

Das Messgerät wird also mit zumindest einem Teil, vorzugsweise mit einer Einführseite des Messgeräts, in den Behälter eingeführt und entnimmt innerhalb des Behälters eine der in dem Behälter gelagerten Messmitteleinheiten und wird mit der entnommenen Messmitteleinheit wieder aus dem Behälter herausgeführt. Der Behälter ist somit im Gegensatz zum Stand der Technik kein Dispenser, denn er gibt keine Messmitteleinheit aus. Erfindungsgemäß dringt dagegen das Messgerät in den Behälter ein und holt sich eine Messmitteleinheit heraus.

Wenngleich im Folgenden die Erfindung beispielhaft anhand eines Blutzuckerspiegelmessgeräts beschrieben wird, ist die Erfindung nicht auf das Beladen von Blutzuckerspiegelmessgeräten beschränkt, sondern kann bei beliebigen Messgeräten eingesetzt werden, die mit einer Messmitteleinheit beladen werden.

Es sei hier ferner angemerkt, dass das Verfahren nicht darauf beschränkt ist, dass der Behälter ortsfest ist und ausschließlich das Messgerät auf den Behälter zu bewegt wird. Das Einführen und Herausführen zumindest einen Teils des Messgeräts in den Behälter ist als relative Bewegung zwischen Messgerät und Behälter zu verstehen, sodass sich sowohl Messgerät als auch Behälter aufeinander zu bewegen können oder sich der Behälter auf das Messgerät zu bewegen kann während das Messgerät ortsfest bleibt oder umgekehrt sich das Messgerät auf den ortsfesten Behälter zu bewegen kann.

Das erfindungsgemäße Verfahren kombiniert den Vorteil eines kompakten Messgeräts der Dosen-Systeme und das hygienische, und sichere Beladen von integrierten Systemen. Gegenüber der EP 2 157 428 A1 hat die Erfindung den Vorteil, dass sich mit nur einem motorisch außerordentlich einfach durchzuführenden Handgriff ohne jegliche zusätzliche Betätigung eines Schiebers eine relativ kleine Messmitteleinheit sicher und hygienisch in das Messgerät laden lässt.

Der Behälter ist dabei vorzugsweise vollkommen separat vom Messgerät ausgestaltet und kann mit dem Messgerät manuell zusammengeführt werden. Es ist allerdings auch eine mechanische Kopplung von Behälter und Messgerät über eine Bewegungsführung wie etwa eine Schiene denkbar. Das Zusammenführen, Einführen und Herausführen wird vorzugsweise manuell durchgeführt, wobei sich in einer Hand das Messgerät und in der anderen Hand der Behälter gehalten werden. Die Hände müssen dabei nur aufeinander zu und nach der Entnahme wieder auseinandergeführt werden. Es bedarf dabei nicht einmal einer gesunden Fingermotorik.

Vorzugsweise weist das Verfahren ferner die folgenden Schritte auf:

- Verschieben der Mehrzahl von in dem Behälter befindlichen Messmitteleinheiten innerhalb des Behälters durch das Einführen des zumindest einen Teils des Messgeräts gegen eine erste Rückstellkraft und
- Halten genau einer in dem Behälter befindlichen Messmitteleinheit in einer Entnahmeposition, sodass diese Messmitteleinheit beim Verschieben der anderen Messmitteleinheiten nicht mitverschoben wird und in der Entnahmeposition mittels des Messgeräts entnommen wird.

Die Entnahmeposition ist dabei vorzugsweise fest durch den Behälter definiert, sodass ein an dem zumindest einen Teil des Messgeräts angeordnetes Entnahmemittel beim Einführen genau an die Entnahmeposition greift. Durch das Verschieben der anderen Messmitteleinheiten ist die an der Entnahmeposition gehaltene Messmitteleinheit von den anderen Messmitteleinheiten separiert und vorzugsweise in dem Entnahmemittel des Messgeräts sicher einklemmbar.

Vorzugsweise werden ferner die in dem Behälter befindlichen Messmitteleinheiten innerhalb des Behälters mittels der ersten Rückstellkraft während des Herausführens des zumindest einen Teils des Messgeräts zurückverschoben. Dadurch wird in einfacher Weise wieder eine Ausgangsposition für die folgende Beladung erreicht.

Vorzugsweise werden ferner die verschobenen Messmitteleinheiten innerhalb des Behälters mittels einer zweiten Rückstellkraft verrückt, sodass eine der nicht entnommenen Messmitteleinheiten in die Entnahmeposition rückt. Dadurch wird in einfacher Weise die nächste Messmitteleinheit für die folgende Beladung in die Entnahmeposition positioniert.

Vorzugsweise erfolgt das Verrücken im Wesentlichen orthogonal zum Verschieben. Dies ist besonders dann von Vorteil, wenn der Behälter einen Stapel von streifenförmigen Messmitteleinheiten in einer Magazineinheit aufweist. Die erste Rückstellkraft kann vorzugsweise die Magazineinheit selbst innerhalb des Behälters entlang einer ersten Achse bewegen. Entlang dieser ersten Achse wird auch vorzugsweise das Messgerät in den Behälter eingeführt. Entlang einer zur ersten Achse senkrechten zweiten Achse kann die zweite Rückstellkraft vorzugsweise den Stapel von streifenförmigen Messmitteleinheiten innerhalb der Magazineinheit verrücken.

Gemäß einem zweiten Aspekt der Erfindung wird ein Behälter zum Beladen eines Messgeräts mit einer Messmitteleinheit bereitgestellt, wobei

- der Behälter ein einen Innenraum des Behälters begrenzendes Gehäuse aufweist,

wobei das Gehäuse eine Aufnahmeseite zum Einführen zumindest eines Teils des Messgeräts in den Innenraum des Behälters aufweist und
wobei das Gehäuse derart zum Lagern einer Mehrzahl von Messmitteleinheiten geeignet und vorgesehen ist, dass eine der Messmitteleinheiten innerhalb des Innenraums des Behälters mittels des durch die Aufnahmeseite des Behälters eingeführten Messgeräts entnehmbar ist.

Der Behälter ist also dazu eingerichtet, dass das Messgerät zumindest teilweise in den Behälter eingeführt wird und innerhalb des Behälters eine der in dem Behälter gelagerten Messmitteleinheiten entnehmen kann. Der Behälter ist somit im Gegensatz zum Stand der Technik kein Dispenser, denn er gibt keine Messmitteleinheit aus. Erfindungsgemäß ist der Behälter dagegen dazu eingerichtet, dass das Messgerät in den Behälter eindringen und sich eine Messmitteleinheit herausholen kann.

Vorzugsweise weist der Behälter ferner auf:

- eine in dem Gehäuse befindliche Magazineinheit zum Lagern einer Mehrzahl von Messmitteleinheiten,
- eine erste Feder
- und ein Haltemittel, wobei

die Magazineinheit innerhalb des Behälters durch Einführen des zumindest einen Teils des Messgeräts gegen die Rückstellkraft der ersten Feder verschiebbar ist und das Haltemittel dazu ausgestaltet ist, genau eine der in dem Behälter befindlichen Messmitteleinheiten in einer Entnahmeposition zu halten, sodass diese Messmitteleinheit bei einem Verschieben der anderen Messmitteleinheiten nicht mitverschoben wird und in der Entnahmeposition mittels des Messgeräts entnehmbar ist.

Hierdurch wird auf besonders einfache und störunanfällige Weise der Behälter so ausgestaltet, dass das Messgerät in den Behälter eindringen und sich eine Messmitteleinheit herausholen kann.

Vorzugsweise weist der Behälter ferner eine zweite Feder zum Verrücken der nicht entnommenen Messmitteleinheiten innerhalb der Magazineinheit auf, sodass eine der nicht entnommenen Messmitteleinheiten in die Entnahmeposition rückt. Vorteilhaft ist dabei ferner, wenn die Rückstellkraft der zweiten Feder im Wesentlichen orthogonal zur Rückstellkraft der ersten Feder wirkt.

Der Behälter ist vorzugsweise separat vom Messgerät ausgestaltet, kann aber auch über eine Bewegungsführung wie etwa eine Schiene an das Messgerät gekoppelt sein. Eine solche Kopplung ist vorzugsweise lösbar, damit der Behälter in einfacher Weise ausgetauscht werden kann.

Die Magazineinheit ist vorzugsweise dazu ausgestaltet, einen Stapel von streifenförmigen Messmitteleinheiten aufzunehmen, wobei die Messmitteleinheiten in Richtung der Rückstellkraft der zweiten Feder stapelbar sind. Vorzugsweise handelt es sich bei den Messmitteleinheiten um einen Stapel Blutzuckerspiegel-Teststreifen für ein Blutzuckerspiegelmessgerät, der in der Magazineinheit lagerbar ist. Um dem Benutzer den Füllstand der Magazineinheit mit Teststreifen bekannt zu machen, kann der Behälter eine Anzeige aufweisen. Diese kann beispielsweise ein transparenter Gehäuseabschnitt sein, durch den der Füllstand sichtbar ist. Eine Zähleranzeige oder Skalenanzeige ist ebenfalls denkbar.

Die Magazineinheit ist vorzugsweise dazu ausgestaltet, Teststreifen einer Länge von weniger als 26 mm aufzunehmen. Noch bevorzugter ist eine Länge von weniger als 15 mm und am besten ist eine Länge von weniger als 10 mm. Es hat sich nämlich herausgestellt, dass dies für eine zuverlässige Messung ausreicht und es bei Anwendung der vorliegenden Erfindung eines manuellen Handlings der Teststreifen nicht mehr bedarf.

Um zu verhindern, dass die Messmitteleinheiten Feuchtigkeit aufnehmen, kann der Behälter ein Trocknungsmittel aufweisen. Das Trocknungsmittel kann beispielsweise in Form eines Trockenmittelsäckchens im Innern des Behälters vorliegen. Das Material des Gehäuses des Behälters kann auch zumindest teilweise innenseitig selbst ein Trocknungsmittel aufweisen bzw. ein Trocknungsmittel sein.

Die Aufnahmeseite des Behälters weist zum Schutz vor Feuchtigkeit und Verunreinigung eine verschließbare Öffnung auf, die ein Einführen des zumindest einen Teils des Messgeräts erlaubt, aber nach dem Herausziehen wieder verschließbar ist bzw. verschließt. Dies kann eine manuell zu öffnende und zu verschließende Klappe sein, die das Gehäuse möglichst dicht abschließt. Es ist allerdings auch eine mit flexiblen und sich teilweise überlappenden Lamellen selbsttätig verschließende Öffnung denkbar. Dabei werden die Lamellen durch das Messgerät beim Einführen in den Behälter nach innen gedrückt und derart verbogen, dass der zumindest eine Teil des Messgeräts eindringen kann. Beim Herausziehen des Messgeräts nehmen die flexiblen Lamellen selbsttätig wieder ihre überlappende und die Öffnung verschließende Stellung ein.

Vorzugsweise weist der Behälter eine Mehrzahl von im Gehäuse des Behälters gelagerten Messmitteleinheiten auf, und das Gehäuse des Behälters ist vorzugsweise derart verschlossen, dass die darin gelagerten Messmitteleinheiten nicht austauschbar sind. Der Behälter kann dadurch als Verbrauchsartikel besonders dicht.und einfach ausgestaltet sein. Eines unhygienischen Nachfüllens von Messmitteleinheiten ggf. mit ungewollter Befeuchtung der Messmitteleinheiten bedarf es dann nicht mehr. In dieser Ausführungsform sind die Messmitteleinheiten Bestandteil des Behälters.

Gemäß einem dritten Aspekt der vorliegenden Erfindung wird ein Kit bereitgestellt mit:

- einem Messgerät zum Messen unter Verwendung einer Messmitteleinheit und
- einem vom Messgerät separaten Behälter zum Beladen des Messgeräts mit einer Messmitteleinheit,

wobei das Messgerät einen mit einem Entnahmemittel versehenen Einführteil aufweist, die dazu ausgestaltet ist, zur Entnahme einer Messmitteleinheit durch eine Aufnahmeseite eines Gehäuses des Behälters in einen durch das Gehäuse begrenzten Innenraum des Behälters eingeführt zu werden.

Vorzugsweise handelt es sich bei dem Behälter um einen wie oben beschriebenen erfindungsgemäßen Behälter. Das Kit wird vorzugsweise gemäß dem oben beschriebenen erfindungsgemäßen Verfahren zum Beladen des Messgeräts verwendet.

Das Messgerät und der Behälter sind vorzugsweise aufeinander abgestimmt, sodass ein Einführen des Einführteils des Messgeräts in den Behälter möglich ist und das Entnahmemittel des Messgeräts innerhalb des Behälters für die Entnahme richtig positioniert ist.

Vorzugsweise korrespondiert die Form des Einführteils des Messgeräts mit der Form der Aufnahmeseite des Behälters derart, dass der Einführteil des Messgeräts nur in einer definierten Orientierung zum Behälter in das Innere des Behälters eingeführt werden kann. Dies verhindert ein nicht ordnungsgemäßes Zusammenführen von Messgerät und Behälter. Durch eine zumindest teilweise konkav geformte Aufnahmeseite des Behälters und einen entsprechend zumindest teilweise konvex geformten Einführteil des Messgeräts kann auch eine passgenaue Zentrierung beim manuellen Zusammenführen unterstützt werden. Dies kann auch mit einander entsprechenden Schrägflächen oder konischen Flächen erreicht werden.

Vorzugsweise ist eine Messmitteleinheit in dem Entnahmemittel des Messgeräts mittels Einführen des Einführteils des Messgeräts in den Innenraum des Behälters einklemmbar. Beispielsweise kann durch den vorzugsweise manuell aufgebrachten Druck beim Einführen die von dem Haltemittel des Behälters an der Entnahmeposition gehaltene Messmitteleinheit in einen sich verengenden Spalt des Entnahmemittels des Messgeräts gedrückt werden und darin eingeklemmt werden. Dies kann auch durch entsprechende Rastmittel unterstützt werden. Ein sich verengender Spalt kann beispielsweise eine mittels Rückstellkraft nach innen drückende Wandung haben. Diese Wandung kann auch Kontaktköpfe aufweisen, die mittels Rückstellkraft auf Kontaktflächen der Messmitteleinheit elektrisch leitend greifen. Damit bewirkt das Einklemmen zum Halten der Messmitteleinheit gleichzeitig auch einen elektrischen Kontakt zum Anlegen einer Spannung an Elektroden in einem in der Messmitteleinheit befindlichen Sensorbereich.

Im Fall eines mit einem Blutzuckerspiegel-Teststreifen beladenen Blutzuckerspiegelmessgeräts muss ein ausreichender Teil des Teststreifens zugänglich bleiben, damit für die Messung ein Tropfen Körperflüssigkeit, z.B. Blut oder Tränenflüssigkeit, darauf aufgebracht werden kann. Vorzugsweise ragt ein saugfähiger Abschnitt des Teststreifens aus dem Messgerät, auf dem ein Tropfen Körperflüssigkeit aufgebracht werden kann, wobei die Körperflüssigkeit dann durch Kapillarkräfte zu einem Sensorbereich geleitet wird, wo sie mit Glucose-Oxidase reagiert. Ein mit Kontaktflächen versehenes Ende des Teststreifens wird vorzugsweise im Messgerät derart eingeklemmt, dass das Messgerät über die Kontaktflächen eine Spannung im Sensorbereich des Teststreifens anlegen, einen für den Blutzuckerspiegel charakteristischen Strom durch die mit Glucose-Oxidase reagierte Körperflüssigkeit messen und die Glucose-Konzentration anzeigen kann.

Zur hygienischen Entsorgung eines ausgelesenen Teststreifens nach einer Messung kann das Messgerät des Kits auch ein zu betätigendes Freigabemittel aufweisen, sodass der ausgelesene Teststreifen ohne Fingerberührung bei entsprechender Haltung des Messgeräts aus dem Messgerät herausfallen kann. Beispielsweise kann durch ein manuelles Betätigungsmittel eine den Teststreifen einklemmende Federkraft gelöst werden. Das Freigabemittel kann auch ein Auswurfmittel sein, bei dem ein ausgelesener Teststreifen nach einer Messung aktiv durch ein manuelles Betätigungsmittel aus dem Messgerät hinausbefördert wird.

Im Folgenden wird die Erfindung anhand der beiliegenden Figuren näher erläutert. Dabei zeigen

Figuren 1-5 eine perspektivische Ansicht auf eine Ausführungsform des erfindungsgemäßen Kits, das ein Messgerät und einen Behälter aufweist, bei verschiedenen Schritten einer vorteilhaften Ausführungsform des erfindungsgemäßen Verfahrens zum Beladen des Messgeräts mit einer Messmitteleinheit: Zusammenführen (Fig. 1), Einführen (Fig. 2), Herausführen (Fig. 3), Messen (Fig. 4), Auswerfen (Fig. 5);

Fig. 6 eine perspektivische Ansicht auf eine Ausführungsform des erfindungsgemäßen Behälters mit geöffnetem Deckel;

Fig. 7 einen Querschnitt durch eine horizontale Ebene des in Fig. 6 gezeigten Behälters mit geöffnetem Deckel;

Figuren 8 und 9 einen vertikalen Längsschnitt entlang der Bewegungsachse einer ersten vorteilhaften Ausführungsform des erfindungsgemäßen Kits, das ein Messgerät und einen Behälter aufweist, vor dem Einführen (Fig. 8) und bei eingeführtem Messgerät beim Entnehmen (Fig.9);

Figuren 10 und 11 einen vertikalen Längsschnitt entlang der Bewegungsachse einer zweiten vorteilhaften Ausführungsform des erfindungsgemäßen Kits, das ein Messgerät und einen Behälter aufweist, vor dem Einführen (Fig. 10) und bei eingeführtem Messgerät beim Entnehmen (Fig.11).

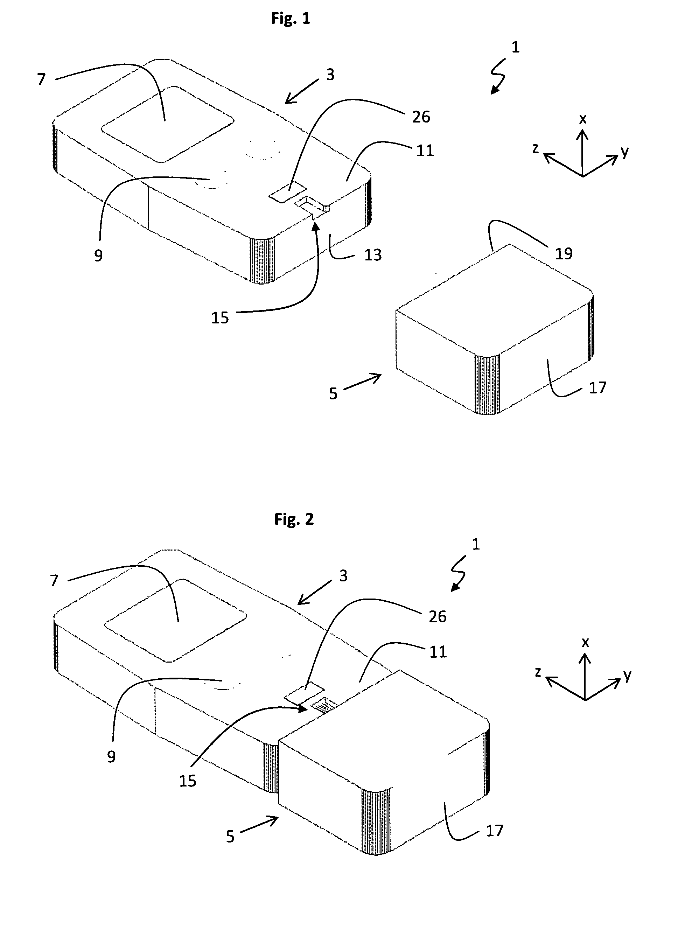

In Figuren 1 bis 5 zeigen ein Kit 1, das ein Messgerät 3 in Form eines Blutzuckerspiegel-Messgeräts und einen vom Messgerät 3 separaten Behälter 5 aufweist. Da das Messgerät 3 und der Behälter 5 frei beweglich zueinander sind, ist es hier sinnvoll, ein rechtshändiges lokales kartesisches Koordinatensystem zu definieren, das in allen Figuren angezeigt ist und fest mit dem Behälter 5 verbunden ist. In der Längsachse des Behälters 5 verläuft die z-Koordinate, in der Querachse verläuft die y-Koordinate und in der Vertikalachse die x-Koordinate. Damit lassen sich alle Bewegungsrichtungen bezüglich des Behälters 5 eindeutig angeben. Eine Bewegungsrichtung $\vec{x}$ des Behälters 5 selbst stellt sich im Hinblick auf nicht mit- bewegte Objekte in dem lokalen Koordinatensystem als eine Bewegung dieser Objekte in Richtung $-\vec{x}$ dar.

Das Messgerät 3 ist in etwa quaderförmig mit abgerundeten Vertikalkanten und ergonomisch für ein Umgreifen mit der Hand angepasst, sodass das Messgerät 3 bequem in einer Hand zu halten ist. Auf der Oberseite weist das Messgerät 3 eine Anzeige 7 zum visuellen Darstellen des Messergebnisses auf. Alternativ oder zusätzlich zur Anzeige 7 wäre auch ein Lautsprecher zur akustischen Wiedergabe des Messergebnisses denkbar, um auch blinden oder sehbehinderten Benutzern die Verwendung des Messgeräts 3 zu vereinfachen. Außerdem befinden sich auf der Oberfläche Bedienelemente 9 in Form von Druckknöpfen, mit denen sich die Messung starten und stoppen lässt und/oder andere Funktionen des Messgeräts 3 aufgerufen bzw. beendet werden können. Das Messgerät selbst kann ggf. auch ganz ohne Anzeige 7 oder Bedienelemente 9 ausgeführt sein und über eine Kommunikationsschnittstelle verfügen, über die ein anderes Gerät das Messgerät steuert und Messergebnisse ausliest, sodass auf dem anderen Gerät eine Anzeige oder Ausgabe des Messergebnisses möglich ist.

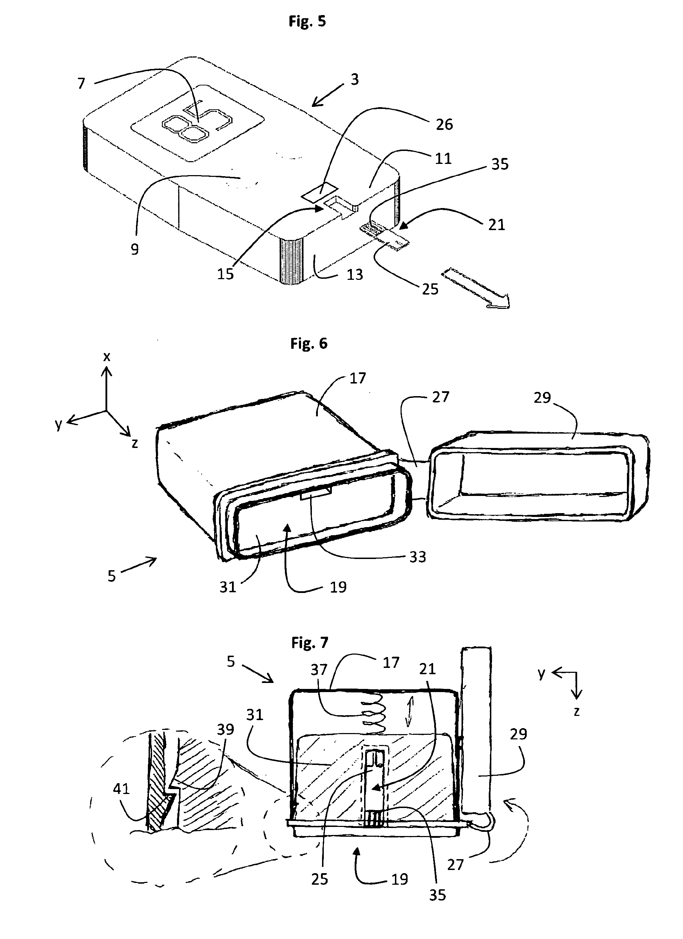

Ein dem Behälter 5 zugewandter Teil des Messgeräts 3 wird im Folgenden als Einführteil 11 bezeichnet, der in den Behälter 5 eingeführt werden kann. Der Einführteil 11 des Messgeräts 3 hat an der dem Behälter 5 zugewandten Seite eine Einführseite 13, an deren oberer Kante sich mittig ein Entnahmemittel 15 befindet. Der Behälter 5 weist ein Gehäuse 17 auf, das einen Innenraum des Behälters 5 nach außen hin begrenzt. Die dem Messgerät 3 zugewandte Seite des Behälters 5 ist eine Aufnahmeseite 19, die mit der Form des Einführteils 11 des Messgeräts 3 (siehe Fig. 6) derart korrespondiert, dass der Einführteil 11 des Messgeräts 3 nur in einer definierten Orientierung zum Behälter 5 in das Innere des Behälters 5 eingeführt werden kann. Dies ist hier wie gezeigt der Fall, wenn die Vertikalachse des Messgeräts in x-Richtung verläuft, die Querachse in y-Richtung verläuft sowie der Einführteil 11 des Messgeräts 3 und die Aufnahmeseite 19 des Behälters 5 in z-Richtung aufeinander zu bewegt werden.

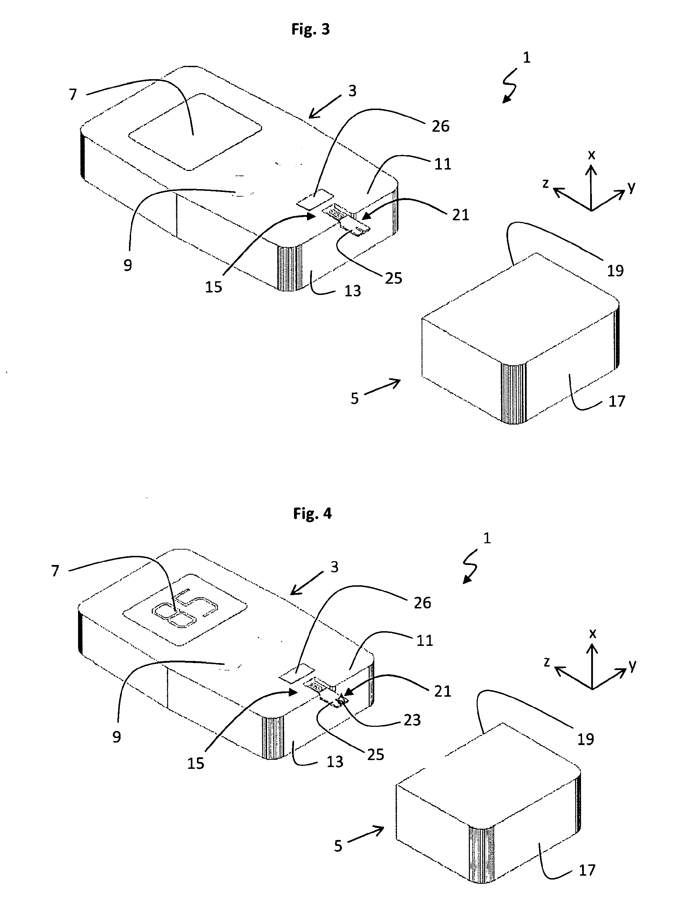

In Fig. 1 ist ein Zustand beim Zusammenführen von Messgerät 3 und Behälter 5 entlang der z-Achse gezeigt bevor sich Messgerät 3 und Behälter 5 berühren. In Figur 2 haben das Messgerät 3 und der Behälter 5 bereits Kontakt und der Einführteil 11 des Messgeräts 3 wird gerade in den Behälter 5 eingeführt. In Figur 3 ist das Messgerät 3 bereits aus dem Behälter 5 wieder herausgeführt und nach dem Entnehmen einer Messmitteleinheit 21 in Form eines Blutzucker-Teststreifens mit dieser Messmitteleinheit 21 im Entnahmemittel 15 beladen. In Fig. 4 ist ein Tropfen Blut 23 bereits auf einem saugfähigen aus dem Messgerät 3 herausragenden Abschnitt 25 aufgetragen und die Messung durchgeführt worden. Das Messgerät zeigt als Messergebnis auf der Anzeige 7 einen Wert von 85 mg/dl an, was einer molaren Glucose-Konzentration im Blut von 4,72 mmol/l entspricht.

In Fig. 5 ist nur das Messgerät 3 gezeigt, wobei ein Freigabemittel, das einen Knopf 26 aufweist, betätigt wurde, was die Messmitteleinheit 21 in dem Entnahmemittel 15 gelöst hat, sodass die verbrauchte Messmitteleinheit 21 nach der Messung durch entsprechende Drehung des Messgeräts 3 ohne manuelle Berührung der verbrauchten Messmitteleinheit 21 "hinausgeschüttet" werden kann. Das Freigabemittel kann auch als aktives Auswurfmittel ausgestaltet sein, mit dem ein ausgelesener Teststreifen nach einer Messung aktiv mittels Betätigung des Freigabemittels aus dem Messgerät 3 hinausbefördert wird.

Fig. 6 zeigt eine Ausführungsform eines erfindungsgemäßen Behälters 5, der an der Aufnahmeseite 19 einen über eine flexible Verbindung 27 angelenkten und geöffneten Deckel 29 aufweist. Der Deckel 29 kann den vom Gehäuse 17 begrenzten Innenraum des Behälters 5 an der Aufnahmeseite 19 vorzugsweise luftdicht abschließen, sodass der Innenraum insbesondere vor Luftfeuchtigkeit geschützt ist. Da bei geöffnetem Deckel 29 Luftfeuchtigkeit eintreten kann, weist der Behälter 5 vorzugsweise ein Trocknungsmittel wie etwa Kieselgel auf, um zu verhindern, dass darin gelagerte Messmitteleinheiten Feuchtigkeit aufnehmen. Das Trocknungsmittel kann beispielsweise in Form eines Trockenmittelsäckchens im Innern des Behälters vorliegen. Das Material des Gehäuses 17 und/oder Deckels 29 des Behälters 5 kann auch zumindest teilweise innenseitig selbst ein Trocknungsmittel aufweisen bzw. ein Trocknungsmittel sein.

Innerhalb des Gehäuses 17 des Behälters 5 befindet sich eine Magazineinheit 31, auf deren Außenseite man bei geöffnetem Deckel 29 durch die Aufnahmeseite 19 blicken kann. An der Außenseite weist die Magazineinheit 31 mittig oben einen Öffnungsschlitz 33 auf, durch den eine Messmitteleinheit 21 in Form eines Blutzuckerspiegel-Teststreifens der Länge nach hindurch passt.

Fig. 7 zeigt einen Querschnitt durch eine horizontale Ebene des in Fig. 6 gezeigten Behälters 5 mit geöffnetem Deckel 29. In der Magazineinheit 31 sind in x-Richtung gestapelte Messmitteleinheiten 21 gelagert. Die gelagerten Messmitteleinheiten 21 sind Blutzuckerspiegel-Teststreifen mit einer Länge in z-Richtung von weniger als 15 mm, einer Breite in y-Richtung von weniger als 5 mm und einer Dicke in x-Richtung von weniger als 0,5 mm. Ein frischer Stapel umfasst mindestens 20 Teststreifen, wobei der oberste Teststreifen des Stapels in einer Entnahmeposition direkt hinter dem Öffnungsschlitz 33 der Magazineinheit 31 liegt.

Die Messmitteleinheiten 21 weisen jeweils einen Sensorbereich 35 auf, der sich an dem zum Öffnungsschlitz 33 der Magazineinheit 31 weisenden Ende befindet. Ferner weisen die Messmitteleinheiten 21 an dem vom Öffnungsschlitz 33 der Magazineinheit 31 weg weisenden Ende einen saugfähigen Abschnitt 25 zum Aufnehmen eines Tropfens 23 Körperflüssigkeit auf.

Die Magazineinheit 31 ist innerhalb des Gehäuses 17 in negative z-Richtung gegen eine erste Feder 37 verschiebbar. Wenngleich die erste Feder 37 als Spiralfeder dargestellt ist, kann die erste Feder 37 auch eine Blattfeder oder jegliches geeignete Federmittel sein. Wie im vergrößerten Auszug dargestellt, ist die Magazineinheit 31 durch ein Rastmittel 39 im Gehäuse 17 eingerastet, sodass die erste Feder 37 die Magazineinheit 31 gegen einen Anschlag 41 drückt. Der Anschlag 41 hält damit die entlang der z-Achse bewegliche Magazineinheit 31 im Gehäuse 17. In dieser gezeigten Ausführungsform ist die Magazineinheit 31 nicht aus dem Gehäuse 17 herausnehmbar und somit nicht wieder auffüllbar. Der Behälter 5 ist damit ein Verbrauchsartikel. Eines unhygienischen Nachfüllens von Messmitteleinheiten 21 ggf. mit ungewollter Befeuchtung der Messmitteleinheiten 21 bedarf es dann nicht mehr. In dieser Ausführungsform sind die Messmitteleinheiten 21 Bestandteil des erfindungsgemäßen Behälters 5.

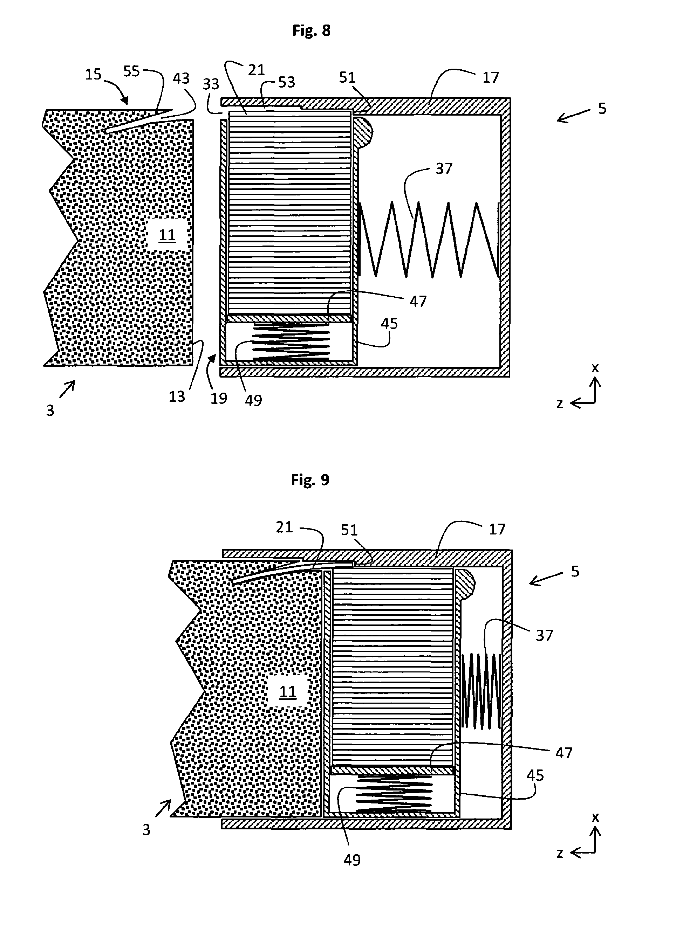

Fig. 8 zeigt einen vertikalen Längsschnitt in der xz-Ebene durch den Behälter 5 kurz vor dem Einführen eines Messgeräts 3. Das Messgerät 3 weist einen Einführteil 11 auf, der an der dem Behälter 5 zugewandten Seite eine Einführseite 13 hat. An der oberen Kante der Einführseite 13 befindet sich ein Entnahmemittel 15. Das Entnahmemittel 15 weist einen Einführschlitz 43 auf, durch den ein Blutzuckerspiegel-Teststreifen der Länge nach eingesteckt werden kann. Wenn die Einführseite 13 zum Beladen des Messgeräts 3 mit einer Messmitteleinheit 21 bestimmungsgemäß vor dem geöffneten Behälter 5 positioniert wird, liegt der Einführschlitz 43 direkt am Öffnungsschlitz 33 der Magazineinheit 31 des Behälters 5.

Die Messmitteleinheiten 21 sind in der Magazineinheit 31 derart auf einer in x-Richtung innerhalb eines Magazinrahmens 45 beweglichen Stützplatte 47 gestapelt, sodass eine unter der Stützplatte 47 angeordnete zweite Feder 49 die Stützplatte 47 und damit den Stapel Messmitteleinheiten 21 nach oben drückt. Wenngleich die zweite Feder 49 als Spiralfeder dargestellt ist, kann die zweite Feder 49 auch eine Blattfeder oder jegliches geeignete Federmittel sein. Die zweite Feder 49 stützt sich unten gegen den Magazinrahmen 45 ab, der in negative z-Richtung gegen die erste Feder 37 beweglich im Gehäuse 17 gelagert ist.

In der in Fig. 8 gezeigten Ausgangsposition nimmt die Magazineinheit 31 eine in z-Richtung maximale Position ein. An der Oberseite des Gehäuses 17 ist innenseitig ein Haltemittel 51 in Form einer Anschlagskante angeordnet, die hinter die oberste Messmitteleinheit 21 des Stapels greift, die sich in einer Entnahmeposition befindet. Vorzugsweise erstreckt sich das Haltemittel 51 um 50% bis 90% der Dicke einer Messmitteleinheit 21 in den Innenraum des Behälters 5. Damit wird sichergestellt, dass das Haltemittel 51 die oberste Messmitteleinheit 21 sicher umgreift, aber nicht die zweitoberste Messmitteleinheit 21 mit umgreift. Damit die oberste Messmitteleinheit 21 besser entnommen werden kann, weist der Behälter 5 oberhalb der obersten Messmitteleinheit 21 aufnahmeseitig einen Eingriffsraum 53 auf, der sich vorzugsweise über höchstens die vorderen zwei Drittel der Länge der obersten Messmitteleinheit 21 erstreckt.

Fig. 9 zeigt die Situation bei in den Behälter 5 eingeführtem Messgerät 3 beim Entnehmen der obersten Messmitteleinheit 21. Das Messgerät 3 wurde manuell mit seiner Einführseite 13 durch die Aufnahmeseite 19 gegen die Außenseite der Magazineinheit 31 gedrückt. Durch manuelles Halten des Gehäuses 17 hat sich die Magazineinheit 31 gegen die erste Feder 37 in negative z-Richtung tiefer in das Gehäuse 17 verschoben. In der in Fig. 9 gezeigten Endposition nimmt die Magazineinheit 31 eine in z-Richtung minimale Position ein, in der die erste Feder 37 maximal gestaucht ist. Während des Verschiebens wurde allerdings die oberste Messmitteleinheit 21 durch das Haltemittel 51 in der Entnahmeposition gehalten. Da sich der Öffnungsschlitz 33 der Magazineinheit 31 um höchstens 10% bis 50 % der Dicke einer Messmitteleinheit 21 unterhalb der obersten Messmitteleinheit 21 erstreckt, kann sich der Öffnungsschlitz 33 nach hinten bewegen während die oberste Messmitteleinheit 21 in der Entnahmeposition gehalten wird. Die oberste Messmitteleinheit 21 wird also passiv durch den Öffnungsschlitz 33 gefädelt.

Eine Zunge 55 des Entnahmemittels 15 des Messgeräts 3 greift beim Einführen in den Eingriffsraum 53 ein und führt die oberste Messmitteleinheit 21 in den sich schräg nach innen in das Messgerät erstreckenden Einführschlitz 43 des Entnahmemittels 15. Dort wird der Sensorbereich 35 der obersten Messmitteleinheit 21 eingeklemmt, sodass das Messgerät 3 mit der eingeklemmten Messmitteleinheit 21 wieder aus dem Behälter 5 herausgezogen werden kann. Beim Herausziehen des Behälters 5 in z-Richtung folgt die Magazineinheit 31 in z-Richtung mittels der Rückstellkraft der ersten Feder 37. Sobald die Magazineinheit 31 wieder die in z-Richtung maximale Position erreicht hat (wie in Fig. 8), verrückt die zweite Feder 49 die noch gelagerten Messmitteleinheiten 21 um eine Position nach oben. Die zuvor zweitoberste Messmitteleinheit 21 ist nach der Entnahme einer Messmitteleinheit 21 die oberste Messmitteleinheit 21 und rückt in die Entnahmeposition.

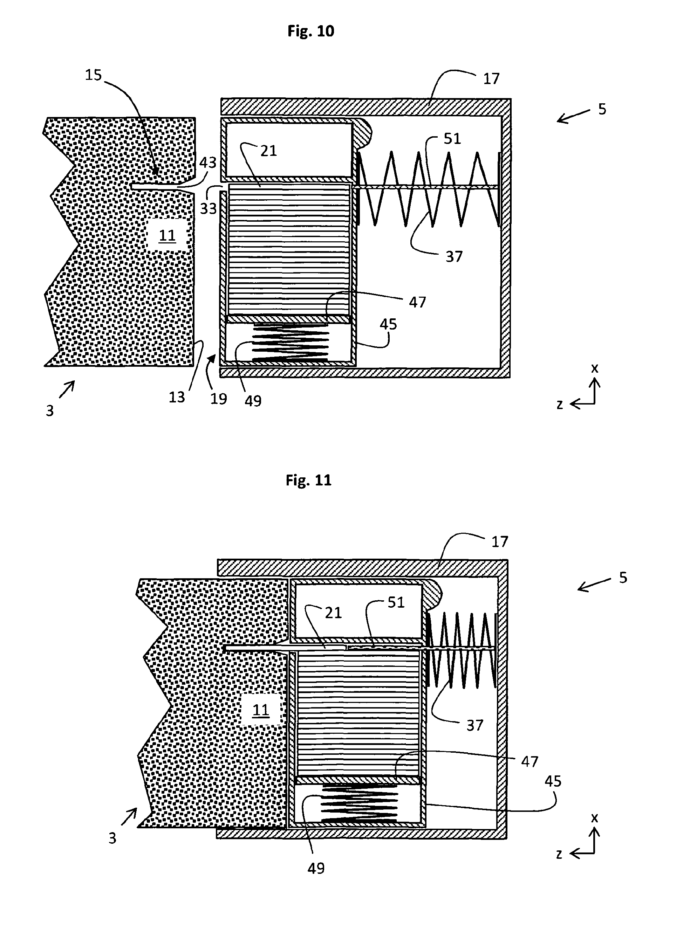

Figuren 10 und 11 zeigen eine Alternative zu der in den Figu-ren 8 und 9 gezeigten Ausführungsform. Hierbei befindet sich die Entnahmeposition nicht am Gehäuse 17, sondern beabstandet davon. Dementsprechend befindet sich der Öffnungsschlitz 33 der Magazineinheit 31 auch tiefer. Dies hat den Vorteil, dass bereits auf dem Markt erhältliche Messgeräte, die einen seitlich entsprechend tiefer gelegenen Einführschlitz 43 aufweisen, mit dem Behälter 5 beladen werden können. Nachteilig gegenüber der in Figuren 8 und 9 gezeigten Ausführungsform ist allerdings, dass nicht die ganze Höhe des Innenraums des Behälters 5 als Füllhöhe für Messmitteleinheiten 21 zur Verfügung steht.

Als Haltemittel 51 dient hier ein bezüglich des Gehäuses 17 ortsfester "passiver Schieber", der sich gegen das Gehäuse 17 abstützt. Das Haltemittel 51 hält die oberste Messmitteleinheit 21 in der Entnahmeposition während die anderen Messmitteleinheiten 21 nach hinten geschoben werden. Hier bedarf es keines speziellen Eingriffsraums oberhalb der obersten Messmitteleinheit 21, da durch die tiefere Entnahmeposition bereits genug Platz zum Entnehmen der obersten Messmitteleinheit 21 zur Verfügung steht.

Durch die vorliegende Erfindung kann eine Messmitteleinheit 21 nur durch das Zusammenführen von Messgerät 3 und Behälter 5 auf außerordentlich einfache, sichere, hygienische und wirtschaftliche Weise mit einer Messmitteleinheit 21 beladen werden.

Bezugszeichenliste

1    Kit

3    Messgerät

5    Behälter

7    Anzeige

9    Bedienelemente

11    Einführteil des Messgeräts

13    Einführseite des Messgeräts

15    Entnahmemittel des Messgeräts

17    Gehäuse

19    Aufnahmeseite

21    Messmitteleinheit

23    Tropfen Blut

25    saugfähiger Abschnitt der Messmitteleinheit

26    Knopf

27    flexible Verbindung

29    Deckel

31    Magazineinheit

33    Öffnungsschlitz

35    Sensorbereich der Messmitteleinheit

37    erste Feder

39    Rastmittel

41    Anschlag

43    Einführschlitz

45    Magazinrahmen

47    Stützplatte

49    zweite Feder

51    Haltemittel

53    Eingriffsraum

55    Zunge des Messgeräts

Patentansprüche

1.    Verfahren zum Beladen eines Messgeräts (3) mit einer Messmitteleinheit (21) aufweisend die Schritte:

- Einführen zumindest eines Teils (11) des Messgeräts (3) in einen eine Mehrzahl von Messmitteleinheiten (21) enthaltenden Behälter (5),
- Entnehmen einer der Messmitteleinheiten (21) mittels des Messgeräts (3) innerhalb des Behälters (5),
- Herausführen zumindest einen Teils (11) des Messgeräts (3) aus dem Behälter (5) mit der entnommenen Messmitteleinheit (21).

2.    Verfahren nach Anspruch 1, ferner aufweisend die Schritte:

- Verschieben der Mehrzahl von in dem Behälter (5) befindlichen Messmitteleinheiten (21) innerhalb des Behälters (5) durch das Einführen des zumindest einen Teils (11) des Messgeräts (3) gegen eine erste Rückstellkraft und

- Halten genau einer in dem Behälter (5) befindlichen Messmitteleinheit (21) in einer Entnahmeposition, sodass diese Messmitteleinheit (21) beim Verschieben der anderen Messmitteleinheiten (21) nicht mitverschoben wird und in der Entnahmeposition mittels des Messgeräts (5) entnommen wird.

3. Verfahren nach Anspruch 2, wobei ferner die in dem Behälter (5) befindlichen Messmitteleinheiten (21) innerhalb des Behälters (5) mittels der ersten Rückstellkraft zurückverschoben werden während des Herausführens des zumindest einen Teils (11) des Messgeräts (3).

4. Verfahren nach Anspruch 2 oder 3, wobei ferner die verschobenen Messmitteleinheiten (21) innerhalb des Behälters (5) mittels einer zweiten Rückstellkraft verrückt werden, sodass eine der nicht entnommenen Messmitteleinheiten (21) in die Entnahmeposition rückt.

5. Verfahren nach Anspruch 4, wobei das Verrücken im Wesentlichen orthogonal zum Verschieben erfolgt.

6. Behälter (5) zum Laden einer Messmitteleinheit (21) in ein Messgerät (3), wobei

- der Behälter (5) ein einen Innenraum des Behälters (5)
begrenzendes Gehäuse (17) aufweist,
wobei das Gehäuse (17) eine Aufnahmeseite (19) zum Einführen zumindest eines Teils (11) eines Messgeräts (3) in den Innenraum des Behälters (5) aufweist und
wobei das Gehäuse (17) derart zum Lagern einer Mehrzahl von Messmitteleinheiten (21) geeignet und vorgesehen ist, dass eine der Messmitteleinheiten (21) innerhalb des Innenraums des Behälters (5) mittels eines durch die Aufnahmeseite (19) des Behälters (5) eingeführten Messgeräts (3) entnehmbar ist.

7. Behälter nach Anspruch 6, ferner aufweisend:

- eine in dem Gehäuse (17) befindliche Magazineinheit (31) zum Lagern einer Mehrzahl von Messmitteleinheiten (21),
- eine erste Feder (37),
- und ein Haltemittel (51), wobei
die Magazineinheit (31) innerhalb des Behälters (5) durch Einführen des zumindest einen Teils (11) eines Messgeräts (3) gegen die Rückstellkraft der ersten Feder (37) verschiebbar ist und das Haltemittel (51) dazu ausgestaltet ist, genau eine der in dem Behälter (5) befindlichen Messmitteleinheiten (21) in einer Entnahmeposition zu halten, sodass diese Messmitteleinheit (21) bei einem Verschieben der anderen Messmitteleinheiten (21) nicht mitverschoben wird und in der Entnahmeposition mittels eines Messgeräts (3) entnehmbar ist.

8. Behälter nach Anspruch 7, ferner aufweisend eine zweite Feder (49) zum Verrücken der nicht entnommenen Messmitteleinheiten (21) innerhalb der Magazineinheit (31), sodass eine der nicht entnommenen Messmitteleinheiten (21) in die Entnahmeposition rückt.

9. Behälter nach Anspruch 8, wobei die Rückstellkraft der zweiten Feder (49) im Wesentlichen orthogonal zur Rückstellkraft der ersten Feder (37) wirkt.

10. Behälter nach einem der Ansprüche 6 bis 9, wobei der Behälter (5) separat von einem Messgerät (3) ausgestaltet ist.

11. Behälter nach einem der Ansprüche 8 bis 10, wobei die Magazineinheit (31) dazu ausgestaltet ist, einen Stapel von streifenförmigen Messmitteleinheiten (21) aufzunehmen, wobei die Messmitteleinheiten (21) in Richtung der Rückstellkraft der zweiten Feder (49) stapelbar sind.

12. Behälter nach einem der Ansprüche 6 bis 11, wobei der Behälter (5) eine Mehrzahl von im Gehäuse (17) des Behälters (5) gelagerten Messmitteleinheiten (21) aufweist und das Gehäuse (17) des Behälters (5) derart verschlossen ist, dass die darin gelagerten Messmitteleinheiten (21) nicht austauschbar sind.

13. Behälter nach einem der Ansprüche 6 bis 12, wobei der Behälter (5) eine Mehrzahl von im Gehäuse (17) des Behälters (5) gelagerten streifenförmigen Messmitteleinheiten (21) aufweist, die kürzer als 27 mm, vorzugsweise kürzer als 15 mm und idealerweise kürzer als 10 mm sind.

14. Kit aufweisend

- ein Messgerät (3) zum Messen unter Verwendung einer Messmitteleinheit (21) und
- einen vom Messgerät (3) separaten Behälter (5) zum Laden einer Messmitteleinheit (21) in das Messgerät (3), wobei das Messgerät (3) einen mit einem Entnahmemittel (15) versehenen Einführteil (11) aufweist, der dazu ausgestaltet ist, zur Entnahme einer Messmitteleinheit (21) durch eine Aufnahmeseite (19) des Behälters (5) in einen durch das Gehäuse (17) begrenzten Innenraum des Behälters (5) eingeführt zu werden.

15. Kit nach Anspruch 12, wobei die Form des Einführteils (11) des Messgeräts (3) mit der Form der Aufnahmeseite (19) des Behälters (5) derart korrespondiert, dass der Einführteil (11) des Messgeräts (3) nur in einer definierten Orientierung zum Behälter (5) in den Innenraum des Behälters (5) eingeführt werden kann.

16. Kit nach Anspruch 12 oder 13, wobei mittels Einführen des Einführteils (11) des Messgeräts (3) in den Innenraum des Behälters (5) eine Messmitteleinheit (21) in dem Entnahmemittel (15) einklemmbar ist.

**Fig. 1**

**Fig. 2**

**Fig. 3**

**Fig. 4**

**Fig. 5**

**Fig. 6**

**Fig. 7**

**Fig. 8**

**Fig. 9**

**Fig. 10**

**Fig. 11**

Europäisches
Patentamt

European
Patent Office

Office européen
des brevets

## EUROPÄISCHER TEILRECHERCHENBERICHT

nach Regel 62a und/oder 63 des Europäischen Patentübereinkommens. Dieser Bericht gilt für das weitere
Verfahren als europäischer Recherchenbericht.

**Nummer der Anmeldung**

EP 12 00 0258

### EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (IPC) |
|---|---|---|---|
| X | US 2006/266765 A1 (PUGH JERRY T [US]) 30. November 2006 (2006-11-30) * Absätze [0055] - [0059]; Abbildungen 11A-11D * ----- | 1,6,10, 12,13 | INV. G01N33/487 |
| X | US 2005/186162 A1 (SATO YOSHIHARU [JP]) 25. August 2005 (2005-08-25) | 1,6, 10-12 | |
| Y | * Zusammenfassung; Abbildungen 1,7A-7D * ----- | 2-5,7-9 | |
| Y | US 2008/021296 A1 (CREAVEN JOHN P [US]) 24. Januar 2008 (2008-01-24) * Zusammenfassung; Abbildungen 1-2d * ----- | 2-5,7-9 | |
| X | US 2007/080093 A1 (BOOZER BRAD [US] ET AL) 12. April 2007 (2007-04-12) * Zusammenfassung; Abbildungen * ----- | 6,10 | |

RECHERCHIERTE
SACHGEBIETE (IPC)

G01N

### UNVOLLSTÄNDIGE RECHERCHE

Die Recherchenabteilung ist der Auffassung, daß ein oder mehrere Ansprüche, den Vorschriften des EPÜ nicht entspricht bzw. entsprechen, so daß nur eine Teilrecherche (R.62a, 63) durchgeführt wurde.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| Den Haag | 14. Juni 2012 | Wilhelm, Jörg |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
...................................................................
& : Mitglied der gleichen Patentfamilie, übereinstimmendes
    Dokument

EPO FORM 1503 03.82 (P04E09)

**Europäisches Patentamt**

**European Patent Office**

**Office européen des brevets**

**UNVOLLSTÄNDIGE RECHERCHE
ERGÄNZUNGSBLATT C**

```
Vollständig recherchierbare Ansprüche:
     1-13

Nicht recherchierte Ansprüche:
     14-16

Grund für die Beschränkung der Recherche:

Ein Behälter nach Anspruch 6 und ein den Behälter beinhaltendes Kit nach
Anspruch 14 sind keine in Beziehung stehenden Erzeugnisse im Sinne von
Regel 43(2)(a) EPÜ, siehe T671/06.
Gemäß Hilfsantrag des Anmelders wurde die Recherche auf die Ansprüche
1-13 beschränkt.
```

**EP 2 618 144 A1**

**ANHANG ZUM EUROPÄISCHEN RECHERCHENBERICHT
ÜBER DIE EUROPÄISCHE PATENTANMELDUNG NR.**

EP 12 00 0258

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-06-2012

| Im Recherchenbericht angeführtes Patentdokument | Datum der Veröffentlichung | Mitglied(er) der Patentfamilie | Datum der Veröffentlichung |
|---|---|---|---|
| US 2006266765 A1 | 30-11-2006 | US 2006266765 A1<br>US 2011198367 A1 | 30-11-2006<br>18-08-2011 |
| US 2005186162 A1 | 25-08-2005 | AU 2003236277 A1<br>CN 1646898 A<br>EP 1494021 A1<br>JP 4143753 B2<br>JP 2003302314 A<br>US 2005186162 A1<br>WO 03085392 A1 | 20-10-2003<br>27-07-2005<br>05-01-2005<br>03-09-2008<br>24-10-2003<br>25-08-2005<br>16-10-2003 |
| US 2008021296 A1 | 24-01-2008 | BR PI0516235 A<br>CA 2584606 A1<br>CN 101061384 A<br>EP 1851539 A1<br>JP 2008518204 A<br>US 2008021296 A1<br>WO 2006047135 A1 | 26-08-2008<br>04-05-2006<br>24-10-2007<br>07-11-2007<br>29-05-2008<br>24-01-2008<br>04-05-2006 |
| US 2007080093 A1 | 12-04-2007 | US 2007080093 A1<br>WO 2007043012 A2 | 12-04-2007<br>19-04-2007 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1352611 B1
- EP 1854738 B1
- US 6508380 B1
- WO 2008125244 A1
- EP 2157428 A1